Europäisches Patentamt

European Patent Office

Office européen des brevets

(11) Publication number: 0 057 107
A2

# (12) EUROPEAN PATENT APPLICATION

(21) Application number: 82300418.9

(22) Date of filing: 27.01.82

(51) Int. Cl.³: **C 12 N 15/00**
C 12 N 5/00, C 12 P 1/00
//A61K39/395, G01N33/54,
C12R1/91

(30) Priority: 28.01.81 GB 8102872
19.06.81 GB 8118902

(43) Date of publication of application:
04.08.82 Bulletin 82/31

(84) Designated Contracting States:
AT BE CH DE FR GB IT LI LU NL SE

(71) Applicant: COATS PATONS PLC
155 St. Vincent Street
Glasgow Scotland(GB)

(72) Inventor: Williamson, Alan Rowe
Laurel House 8 Wyngrave Place Knotty Green
Beconsfield Buckingshamshire(GB)

(72) Inventor: Stimson, William Howard
30 Albert Avenue Bearsden
Dunbartonshire Scotland(GB)

(72) Inventor: Dick, Heather May
Glenside 12 Glenhead Road Lenzie
Dunbartonshire Scotland(GB)

(72) Inventor: Clark, Stuart Airdrie
7 Struma Drive
Clarkston Renfrewshire Scotland(GB)

(74) Representative: Ferguson, James MacKay et al,
Cruikshank & Fairweather 19 Royal Exchange Square
Glasgow, G1 3AE(GB)

(54) Method of manufacturing monoclonal antibodies and cells capable of manufacturing such antibodies.

(57) The invention lies in the field of monoclonal antibodies and other lymphocyte products.

A method of producing a monoclonal antibody and other lymphocyte products comprises fusing together lymphocytes sensitized against a target antigen and tumour-like cells which have been treated to eliminate therein an enzymic activity essential to their survival in the free state to form a mixture of hybrid cells and unfused lymphocytes and tumour-like cells. The mixture is then suspended in a cell culture medium in which the hybrid cells grow and the unfused lymphocytes and tumour-like cells die.

Antibody produced by the hybrid cells may be extracted and used to treat patients suffering from a disease resulting from the presence of the target antigen.

Croydon Printing Company Ltd.

METHOD OF MANUFACTURING MONOCLONAL
ANTIBODIES AND CELLS CAPABLE OF
MANUFACTURING SUCH ANTIBODIES

The subject of this invention is a method
of manufacturing monoclonal antibodies and other
lymphocyte products such as lymphokines and of
manufacturing cells capable of producing such monoclonal
antibodies and other lymphocyte products. The method is
particularly suitable for the manufacture of monoclonal
antibodies for human use but the method is equally
suitable for producing monoclonal antibodies for animal
use particularly farm animals and the larger domestic
pets using materials appropriate to the particular
animals.

The following specification describes the
process mainly with reference to the production of
human monoclonal antibodies and human cells capable
of making such antibodies but what substitutions are
necessary to make monoclonal antibodies and cells for
producing such antibodies for the treatment of animals
and for producing other lymphocyte products will be
readily apparent to those skilled in the art.

Foreign cells or substances (antigens)
entering the body normally cause white blood cells to
respond to their presence. Certain of these blood
cells, known as lymphocytes, recognize particular
determinant groups of the antigen and produce specific
antibodies which are capable of neutralising and
eliminating the foreign substance. Since an antigen
usually bears several such determinants, a number of
antibodies will be formed against each antigenic
component. Each antibody is synthesized by one
lymphocyte type and therefore in any normal situation,
where a body is exposed to a foreign agent many
lymphocytes will respond to produce specific
antibodies against each antigenic component.

There is an unlimited number of antigens to which the body can be exposed so that even among the large number of lymphocytes present in the body there is only a comparatively small number able to produce the antibodies specific to a particular antigen. Thus, although when an antigen is introduced into a body the particular lymphocytes are stimulated to produce antibodies specific for the antigen, it takes an appreciable time which may be numbered in weeks before the lymphocyte population producing the specific antibodies has increased to the stage where the antigen can be removed by the antibodies. The natural delay in the production of specific antibodies by the body can be circumvented by the administration, e.g. by injection, of specific antibodies, which are expected to shorten the course of disease and lead to an early recovery.

Attempts have been made to grow antibody-producing lymphocytes in culture. Unfortunately, antibody-secreting cells can only be grown in culture with difficulty and have a limited life span in the culture medium. The rate of output of antibodies is very low principally because of the short life of the cells and their consequent small amount of growth.

It has already been discovered that myeloma cells of murine origin can be fused with lymphocytes of murine origin and thereupon form hybrid-myeloma or so-called "hybridoma" cells which possess the essential characteristics of both parents, i.e. the specific antibody production capability of the lymphocyte and the relative immortality of the myeloma cell. This has made it possible to select individual hybrid clones, which having a long life and a consequently high growth capability can synthesize large quantities of one kind of antibody to a single antigenic determinant and grow them continuously in culture. To date, it has been difficult to obtain a usable quantity of hybrid cells capable of being employed in antibody production because of the so far inevitable presence in any group of hybridoma cells of free tumour-like cells which have not combined with lymphocytes and because of the inappropriate antibody production by the tumour-like cells. Although hybridomas of murine origin have been found useful in providing antibodies suitable for use in various diagnostic tests employed in hospitals, these antibodies cannot ordinarily be used without risk in the treatment of human patients since they act as antigens in man.

It is an object of the present invention to avoid or minimize one or more of the above disadvantages and in particular to provide inter alia a method of producing in large quantity stable fused-cell hybrids capable of synthesizing monoclonal antibodies against a given "target" antigen without the presence of free tumour-like cells and their products and a method of producing antibodies specific against a given target antigen without the presence of free tumour-like cells.

According to the invention a method of forming cells capable of producing a monoclonal antibody against a target antigen and other lymphocyte products by fusing together tumour-like cells and sensitized lymphocytes to form hybrid cells each comprising at least one lymphocyte and at least one tumour-like cell is characterized in that the tumour-like cells before fusion with the lymphocytes are treated so that at least one enzymic activity therein essential to the survival of the cells in the free state is eliminated and the lymphocytes before fusion with the tumour-like cells are stimulated to become active following which the lymphocytes and the tumour-like cells are brought together in conditions in which some of them fuse to form hybrid cells, the resultant product which contains hybrid cells and free lymphocyte and tumour-like cells which have not fused together is suspended in a cell culture medium in which the hybrid cells continue to grow while the unfused tumour-like cells die as a result of the said treatment of the cells to eliminate an enzymic activity, the unfused lymphocytes which have normally a short life also dying.

An example of another lymphocyte product produced by the method of the invention is lymphokines.

The expression "tumour-like cell" is defined for the purposes of this specification as being any cell exhibiting the immortal characteristics of a tumour cell i.e. the ability to undergo continued division.

For the production of monoclonal antibodies the lymphocytes used are B-lymphocytes but for certain effects e.g. to promote stimulation in certain conditions leucocytes e.g. T-lymphocytes in a predetermined proportion may be mixed with the B-lymphocytes.

To provide tumour-like cells having the characteristic that they cannot survive without a specific enzymic activity normal tumour-like cells may be treated with a cytotoxic substance which blocks the production and/or activity of one or more enzymes essential to the continued survival of the cells in the particular cell culture medium.

A particularly suitable cytotoxic substance for the above purpose has been found to be the biochemical inhibitor diethylpyrocarbonate (DEP) but many other readily available cytotoxic substances are known.

An important advantage of the use of diethylpyrocarbonate is that it is effective for a very wide range of tumour-like cells so that tumour-like cells from practically any source, whether human or non-human may be made enzyme-deficient in a simple and economic manner. A further advantage is that the enzyme deficiency induced is such that the unwanted unfused cells die quickly in any conventional cell culture medium so that the need for special culture media, such as for example HAT (hypoxanthine-aminopterin-thymidine) medium, is avoided.

In practice it is sometimes difficult or not possible to obtain large enough lymphocyte populations sensitized exclusively to a single target antigen and it may therefore be necessary to include the further step of cloning the fused cell hybrid product obtained by the above method in order to achieve a hybrid cell population capable of producing monoclonal antibody i.e. only a single type of antibody which is directed against only the target antigen (or a single antigenic determinant thereof).

To obtain the desired antibody from hybridoma cells produced by the process the hybridoma cells may be stimulated to produce the antibody for example by exposing them to a mitogen or to the target antigen then isolating the antibody from the culture medium in which the hybridoma cells are cultured e.g. by a process of fractionation.

The invention also includes hybrid cells and monoclonal antibody obtained from hybrid cells produced by the method of the present invention.

Sensitized lymphocytes may be obtained from a patient who has recently suffered a natural infection with the target antigen. Alternatively, lymphocytes may be sensitized in vitro, for example, by incubation of lymphocytes with the target antigen and this may be preferred insofar as it provides a sensitized lymphocyte starting material of greater specificity than would otherwise be available.

By means of the present invention it is possible to obtain a hybrid cell which can be readily used to produce large quantities of human monoclonal antibody against a specific target antigen by utilizing cells of human origin only and without any danger of the presence of free active tumour-like cells and unwanted antibodies. The use of cells of non-human origin is totally avoided thereby eliminating the various problems associated with the use of antibodies produced using cells of non-human origin. In particular the possibility of the antibody obtained acting as an antigen in a human patient is very much less than would be expected from the use of xeno-antibodies.

It will also be appreciated that since the present invention makes possible the large-scale production of antibodies against target antigens whether they be of bacterial, viral, neoplastic, or other origin, the invention also makes possible the safe, effective and rapid treatment and/or prophylaxis of patients suffering from or liable to a wide variety of diseases even diseases of which the bacterium or virus has not yet been identified. Also by combining appropriate extra corporeally-produced antibodies with cytotoxic agents malignant tumour cells may be more safely treated since the antibodies, being specific to the malignant tumour cells confine the action of the cytotoxic agents to the tumour cells. Other possibilities include that of long term contraception using human monoclonal antibodies specific for placental proteins e.g. $\beta$-HCG, and prevention of graft and transplant rejection.

A monoclonal antibody produced by the method of the present invention may be mixed with a physiologically acceptable carrier therefor. An example of such a medium is 0.02 molar phosphate buffer pH 7.4 containing 0.147 molar NaCl.

The invention also extends to a diagnostic reagent for use in the qualitative and/or quantitative determination of a target antigen, said reagent comprising a solution of the antibody in a suitable medium which may also be 0.02 molar phosphate buffer pH 7.4 containing 0.147 molar NaCl.

Although tumour-like cells obtained directly from a living donor may be employed as the tumour-like cells for combining with lymphocytes to form the hybrid cells it is generally preferable to employ an established cell line for the method of the present invention since the behaviour of such an established cell line is more predictable and usually results in greater efficiency.

The invention also provides a cell culture system comprising the hybrid cell line of the invention in a nutrient medium. An example of such a nutrient medium is RPMI 1640 containing 10% v/v foetal calf serum.

The tumour-like cells employed in performing the method of the invention may be capable of, may previously have been capable of, or may be derived from cells capable of or having previously been capable of producing antibodies, it is however preferred to use a tumour-like cell which is non-immunoglobulin secreting as this avoids the possibility of production of unwanted antibodies i.e. antibodies other than those specific to the target antigen.

The sensitized lymphocytes may be stimulated by further exposure to said target antigen. Alternatively, the sensitized lymphocytes may be stimulated by exposing them to a mitogen. Examples of mitogens capable of stimulating sensitized lymphocytes are pokeweed mitogen (PWM) and protein A from Staphylococcus Aurus. These mitogens are particularly preferred but other suitable mitogens are bacterial lipopolysaccharide (LPS) and dextran sulphate (DS).

Stimulation of the sensitized lymphocytes is desirably carried out some 3 or 4 days before fusion of the lymphocytes and tumour-like cells is effected. This results in a higher number of fusion events, with resultant increased number of hybrid cell cultures.

The conditions for causing fusion of the lymphocytes and the tumour-like cells may be set by exposing them to a fusion promoting agent. One preferred fusion promoting agent is polyethylene glycol (PEG), for example PEG 1500.

Further preferred features and advantages of the invention will appear from the following Examples. In Example 1 for clarity the several steps of the method of producing human monoclonal antibodies are separately described.

Example 1 - Preparation of Human Monoclonal Antibody-producing Hybrid Cell

A. Isolation of Sensitized Human Lymphocytes.

Peripheral blood was collected from human blood donors who were immune to the anti-RhD antigen of human red cells. The blood was subjected to Ficoll-Isopaque (FI) separation. The cell material collected was found to contain greater than 90% lymphocytes. If desired this material could have been further fractionated in known matter so as to obtain a material containing only B-lymphocytes to the exclusion of other cells e.g. T-lymphocytes and monocytes but this is usually not essential. Various other known methods for isolating lymphocytes could have been employed but the above described method has been found particularly convenient.

B. Stimulation of Sensitized Lymphocytes.

The lymphocytes obtained above were washed twice in serum-free tissue culture medium RPMI 1640 containing 2 mM HEPES, 200 mM glutamine, 5 mM sodium bicarbonate and penicillin 100 IU and streptomycin 100 $\mu$g per ml, and finally suspended in RPMI 1640 medium with 10% human AB serum at a concentration of $10^6$ cells/ml. Aliquots (2ml) of the suspension obtained were pipetted into Linbro plates (flat-bottomed, tissue culture treated, 24 x 3.5 ml well capacity) and Pokeweed mitogen (PWM, 80 $\mu$g per well) added thereto.

The Linbro plate was then placed in an incubator gassed with 95% air and 5% $CO_2$ and humidified and maintained at a temperature of 37°C. On the third/fourth day of stimulation the lymphocytes were used for fusion.

C. Enzyme Blocking.

RPMI 8226 human tumour-like cells (ATCC culture collection deposit number CCL 155 at $2.5 \times 10^5$/ml) were treated with the minimum lethal dose of diethylpyrocarbonate (DEP 0.022% w/V) in serum-free RPMI 1640 medium for 30 minutes at 4°C. The cells were then washed twice in serum-free RPMI 1640 medium. The minimum lethal dose of the DEP was determined by treating samples of the tumour-like cells with a range of concentrations of DEP, the minimum lethal dose being defined as the minimum dose at which no growth was detected after 1 week.

D. Fusion of Tumour-like Cells with Lymphocytes.

The stimulated sensitized lymphocytes of paragraph 1 B above and the enzyme-blocked tumour-like cells of paragraph 1 C above were mixed together in approximately equal numbers ($10^7$ of each/ml) in serum-free RPMI 1640 medium. The cell suspension was centrifuged down and the supernatant liquid decanted. The cell pellet was resuspended, with mixing, in polyethylene glycol (PEG) 1500 (40% w/v, 2 ml). After 2 minutes the suspension was gradually diluted over a period of from 3 to 5 minutes with serum-free RPMI (20 ml). The cell suspension was then spun down and resuspended in RPMI 1640 medium with 10% human AB serum (5ml). This suspension was then incubated at 37°C for from 1 to 3 hours. Following incubation the cell suspension was diluted to a concentration corresponding to $10^5$ enzyme-blocked tumour-like cells per ml with RPMI 1640 medium with 10% human AB serum, human fibrocytes ($10^6$/ml) being added as a feeder layer.(Other material which may be used in the feeder layer include human monocytes, rᵼ thymic epithelial cells, and fibroblast conditioned ᴡedium to provide the necessary growth factors to the hybrid cells). Aliquots (200 $\mu$1) of this cell suspension containing hybrid cells were then placed in microtitre plates and the plates left for two weeks at 37$^O$C, after which samples of the supernatant fluid could be taken for assay.

As an alternative to the above mentioned RPMI 1640 medium various other culture media well known in the art can be employed. One such medium which may be mentioned is Dulbecco's modification of Eagle's minimum essential medium.

E. Cloning of Hybrid Cells and Monoclonal Antibody Production.

Supernatant fluid (100 $\mu$1) was taken from wells in the microtitre plate of Example 1 D and in which significant cell growth had taken place and tested in an Enzyme Immuno-Assay.

A microtitre assay plate was prepared by coating the wells thereof with goat anti-human IgG (GAHI), using a solution (12.5 $\mu$g/ml) in 0.02M Tris HCl, pH9. The GAHI solution (100 $\mu$1) was incubated in the wells overnight at 4$^O$C. The next day the plates were washed twice with Tris wash buffer, once with Tris/Tween buffer and once again with Tris wash buffer. Tris wash buffer contains 0.2 M Tris pH7.4 plus NaCl 0.2M and Tris/tween buffer is Tris wash buffer containing Tween 20 (0.05% v/v). The plates were then shaken dry. Supernatant fluid (100 $\mu$1) from the fusion system wells was added to a corresponding well in a microtitre assay plate and incubated at 18-20$^O$C for 45 minutes. Following incubation the wells were washed three times with Tris/Tween buffer and shaken dry as before.

GAHI - Horse Radish Peroxidase (HRP) enzyme conjugates (linked with glutaraldehyde using a two-stage procedure) was added, using a concentration of 10 $\mu$g/ml

in 0.147M NaCl with 25% normal sheep serum, at 100 $\mu$l per well, and incubated for 30 minutes at 18-20$^{\circ}$C. Following incubation the wells were washed three times with Tris/Tween buffer and the plates shaken dry as before.

Enzyme substrate (100 $\mu$l) containing orthophenylene-diamine (0.4 mg/ml) and hydrogen peroxide (0.32 $\mu$l of a 30% W/V solution/ml) in 0.2 M Phosphate/citrate buffer pH6, was added to each well and incubated for 30 minutes at 18-20$^{\circ}$C in the dark. The reaction was stopped by the addition of 50 $\mu$l of 2M $H_2SO_4$ to each well. The optical density of the supernatant fluid was then read at 492 nm in a spectrometer to obtain an A 492 value which is related to the amount of human immunoglobulin present in the sample taken from a given fusion well.

The cellular contents of positive wells of the microtitre plate of Example 1 D were transferred to Linbro wells to expand the clones. After a further two weeks the supernatant fluids from the wells assayed to confirm specific antibody production, using an appropriate assay, for example an antigen specific Enzyme Immuno-Assay.

It will be appreciated that antibody production by the hybrid cells takes place more or less continuously following fusion. It is normally necessary to clone the hybrid cell population, as described below, in order to obtain a stable hybrid cell line producing monoclonal antibody before large scale production and recovery of antibody is undertaken.

Once a monoclonal antibody-producing hybrid cell clone has been established this may be cultured in any conventional manner, for example, on plates or in spinner vessels, or in animals, supernatant or body fluids removed at suitable intervals and if required replaced by fresh medium, and antibody recovered from the supernatant fluid by the aid of conventional techniques for the isolation of antibody and separation from culture medium constituents.

Suitable techniques for isolation of antibody and separation from culture medium constituents are well known and include for example one or more of electrophoresis, various centrifugation techniques, immunodiffusion, molecular or affinity chromatography, dialysis and fractional precipitation.

Example 2 - Preparation of Hybrid Cells and Human Monoclonal Antibody

The procedure of Example 1 was followed except that in place of RPMI 8226 cell line, there was used in the fusion stage D the cell line derived from the human lymphoblastoid cell line as described hereinabove using the fusion procedure according to Example 1 B. The DEP treatment of the tumour-like cell line (Stage 1 C) was omitted. The cell suspension was finally diluted to a concentration corresponding to $10^6$/ml tumour-like cells in HAT medium.

Example 3 - Pharmaceutical Composition and Method of Treatment

Antibody was prepared by the method of Example 1 or Example 2, against a selected organism or antigen associated with a given disease. After purification by conventional techniques the antibody immunoflobulin or product thereof, as for example, $F(ab^1)_2$ fragments, was made up into a solution in physiological saline (0.147M NaCl) with a selected protein concentration, for example, 3.0% w/v. The solution was then sterilised by filtration and dispensed in a sterile manner into ampoules which were sealed under vacuum.

A patient suffering from a given disease was dosed parenterally with the antibody preparation described in this example in doses appropriate to the infection as determined by existing labora
procedures for the detection and characterisat
infecting agents. The solutions must be steri
with human body fluids, devoid of aggregates a
pyrogen-free.

In some diseases, it is envisaged th
routes of administration as for example, by ae
the respiratory tract or by time release capsu
alimentary tract can be employed, where it is
to obtain a therapeutic effect of the antibody
example at the surface of a mucous membrance w
the infection has come from an organism known
disease-producing effects at such a site. Spe
parenteral administration routes that may be m
include intramuscular, intraperitoneal, subcut
intravenous injection.

Example 4 - Diagnostic Materials and
Immuno-Assay Test

A. Antibody Solution - Antibody obta
from Example 1 was dissolved in 0.02 M Tris:HCl

B. Microtitre Plate - Aliquots (100 $\mu$l) c
the above antibody solution diluted 1:5000 - 1:5
was dispensed into the individual wells of a mi
plate and incubated therein overnight at 4$^O$C.
following day the plates were washed twice wit
wash buffer, once with Tris-Tween buffer, and
with Tris wash buffer and then shaken dry and

C. Antibody Enzyme Conjugate - The
obtained from Example 1 was linked with horse
Peroxidase using glutaraldehyde.

D. Enzyme Immuno-Assay - The above
microtitre plate and conjugate are used to det
presence and content of anti-RhD antigen (cell
by following the method outlined in Example 1.

Other assays for particular antigens and materials therefor are readily obtainable by sensitizing the lymphocytes used in the method of the invention against the particular desired alternative target antigen.

## CLAIMS

1. A method of forming cells capable of producing a monoclonal antibody against a target antigen and other lymphocyte products by fusing together tumour-like cells and sensitized lymphocytes to form hybrid cells each comprising at least one lymphocyte and at least one tumour-like cell is characterized in that the tumour-like cells before fusion with the lymphocytes are treated so that at least one enzymic activity therein essential to the survival of the cells in the free state is eliminated and the lymphocytes before fusion with the tumour-like cells are stimulated to become active following which the lymphocytes and the tumour-like cells are brought together in conditions in which some of them fuse to form hybrid cells, the resultant product which contains hybrid cells and free lymphocyte and tumour-like cells which have not fused together is suspended in a cell culture medium in which the hybrid cells continue to grow while the unfused tumour-like cells die as a result of the said treatment of the cells to eliminate an enzymic activity, the unfused lymphocytes which have normally a short life also dying.

2. The method according to claim 1, characterized in that the lymphocytes are B-lymphocytes mixed in a predetermined proportion with leucocytes.

3. The method according to claim 1, characterized in that the tumour-like cells in which enzymic activity essential to their survival in a particular cell culture medium is absent are obtained by treating normal tumour-like cells with a cytotoxic substance which blocks the production and/or activity of at least one enzyme essential to the continued survival of the cells in the particular cell culture medium.

4. The method according to claim 1, characterized in that the fused-cell hybrid cells isolated from the culture medium are cloned.

5. The method according to claim 1, characterized
in that the hybrid cells are stimulated to produce
the antibody by exposing them to the target antigen.

6. The method according to claim 1, characterized
in that the hybrid cells are stimulated to produce
the antibody by exposing them to a mitogen.

7. The method of claim 1, characterized in
that the tumour-like cells are non-immunoglobulin-
secreting cells.

8. The method according to claim 1, characterized
in that the sensitized lymphocytes are stimulated to
become active by exposure to the target antigen.

9. The method according to claim 1, characterized
in that the sensitized lymphocytes are stimulated to
become active by exposure to a mitogen.

10. Hybrid cells produced by the method
according to claim 1.

11. Monoclonal antibody obtained from cells
produced by the method according to claim 1.